# EUROPEAN PATENT APPLICATION

(11) **EP 4 846 961 A1**
(43) Date of publication of application: **02.12.2026**
(21) Application number: 26182005.4
(22) Date of filing: 29.05.2026
(51) Int. Cl.: A61B 17/80

(54) **TROCHANTER PLATES AND ATTACHMENT PLATES**

(30) Priority: 30.05.2025 US 202563814490 P
(71) Applicant: Stryker European Operations Limited, Carrigtwohill T45H X08 (IE)
(72) Inventor: STUCKI, Katja, 5400 Baden (CH); MURPHY, Daniel, Coachford, Cork, P12 DX51 (IE); CHRISTEN, Alexis, 3360 Herzogenbuchsee (CH); KLOSE, Tilman, 24105 Kiel (DE)
(74) Representative: Maiwald GmbH

(57) **Abstract**

A femur plate includes a shaft portion and a head portion. The shaft portion defining a plurality of holes. The head portion extending from an end of the shaft portion and having two arms configured to at least partially wrap around and attach to trochanters of a femur. Each of the two arms defining a plurality of holes and at least one of the two arms has a suture hook extending from adjacent one of the plurality of holes.

## Description

### CROSS-REFERENCE TO RELATED APPLICATIONS

This application claims the benefit of the filing date of U.S. Provisional Patent Application No. 63/814,490 filed May 30, 2025, the disclosure of which is hereby incorporated herein by reference.

### BACKGROUND

Bone plates for fracture treatment must be firmly fixed to the bone to prevent fragments from moving relative to each other. When fixing a bone plate to a fractured bone, particular care must be taken to ensure proper fixation and to prevent further fracturing of the bone. This becomes difficult in respect to certain types of fractures.

For instance, cases involving trochanteric and subtrochanteric fractures require special care to ensure the fractured bone parts are tightly held together to promote healing. Trochanteric fractures typically occur between the greater trochanteric region and shaft of the femur. When such fractures are treated, surgeons must use bone plates capable of fitting around the contours of the femur. This can be difficult as the size and shape of the trochanteric regions can vary greatly from patient to patient, which may require significant modification (*e.g*., bending) of existing bone plates.

Accordingly, there exists a need for an improved bone plate for treating trochanteric fractures.

### BRIEF SUMMARY

The present disclosure relates to bone plates and attachment plates adapted to conform the contours of a bone and capable of holding fractured bone parts together to promote healing.

One aspect of the present disclosure relates to a bone plate that may have an elongated shaft and arms extending from a first end for the elongated shaft. The elongated shaft may define a width that tapers from the first end to a second end thereof. The elongated shaft may have a top surface defining grooves or indentations extending across the width of the top surface. The indentions may be equally spaced apart from each other along the length of the elongated shaft in a stripe-like pattern with each indentation extending from opposite sides of the elongated shaft. In this manner, the top surface of the elongated shaft may define a textured surface configured to receive various types of cables, wires, sutures, etc.

The elongated shaft may also include a variety of holes such as screw holes and slotted holes. One slotted hole may be located between two of the screw holes along a length of the elongated shaft and have an oval shape. The screw holes may be positioned between the striped pattern defined by the top surface indentions and offset from the longitudinal axis of the elongated shaft. The screw holes may be positioned on opposite sides of the longitudinal axis in an alternate sequence or pattern such that no two sequential screw holes are place on the same side of the elongated shaft. One screw hole may be centered along the longitudinal axis of the elongated shaft at or near the second end of the shaft.

The arms may extend from opposite sides of the first end of the elongate shaft and may include screw holes, suture hooks, and pointed prongs. The screw holes may be equally spaced apart along a length of each arm. The outer sides of the screw holes may be wider than the width of each arm. The suture holes may have a dual hook or dovetail configuration and extend laterally from one arm toward the other arm. The pointed prongs may extend from a screw hole at the terminal end of each arm. The arms may be flexible such that they can conform to the contours of a patient's bone structure to provide at tighter fit therewith.

Another aspect of the present disclosure includes a bone plate for implantation on a femur. The bone plate includes a shaft region defining a plurality of holes and a trochanteric region at one end of a shaft region of the bone plate. The trochanteric region may include two arms capable of forming a concave shape to wrap around curved portions of a patient's bone and define one or more holes for receiving screws that are spaced apart along the two arms. The arms may also include flexible segments disposed between the screw holes of the two arms to facilitate wrapping the arms around bone. In some instances, there may be the same number of screw holes as there are flexible segments. One or both of the arms may include curved hooks at a terminal end of the respective arm and dual-pronged hooks configured to receive suture and wire used to secure the arm to a patient's bone. In other instances, the trochanteric region may include an angled head that extends away from the shaft region. In such instances, the angled head may be obliquely angled with respect to the longitudinal axis of the bone plate and bent upward such that a terminal end of the head portion is elevated above the rest of the bone plate assembly.

The trochanteric and shaft regions may be integral with each other or separate pieces that may be connected or attached to each other such that the overall size of the bone plate assembly may be adjusted as needed. When separate pieces, the trochanteric region may define a recessed slot that is adapted to receive a narrow end portion of the shaft region. The recessed slot may define an elongated gap that aligns with screw holes disposed in the narrow end portion. In such instances, connecting screws may be inserted through the elongated gap and then into the screw holes to secure the trochanteric region to the shaft region.

In another aspect, the present disclose relates to a femur plate that may include a shaft portion and a head portion. The shaft portion may define a plurality of holes. The head portion may extend from an end of the shaft portion and may have two arms configured to at least partially wrap around and attach to trochanters of a femur. Each of the two arms may define a plurality of holes, and at least one of the two arms may have a suture hook extending from adjacent one of the plurality of holes. The shaft portion and the head portion may be separate pieces attached to each other. The shaft portion may be tapered at a terminal end. The head portion may include a receiving portion configured to receive an attachment portion of the shaft portion. The receiving portion may include an elongated slot configured to extend across one or more of the plurality of holes of the shaft portion when the shaft and head portions are assembled together. One or more of the plurality of holes of the shaft and head portions may permit polyaxial placement of screws. The shaft portion and the head portion may be integral. The shaft portion may include an oblong hole disposed between two holes of the holes of the shaft portion. The plurality of holes of the two arms may be configured to receive locking and non-locking screws. The two arms may include bendable sections permitting bending of the arms. One of the two arms may include a prong extending from a terminal end thereof. The prong may be curved and may include a sharpened tip. The prong may be tapered to a pointed tip.

In another aspect, the present disclosure relates to a bone assembly having a base plate and an attachment plate. The base plate may have holes. The attachment plate may have a groove and an elongated slot. The groove may be configured to receive a portion of the base plate, and the elongated slot may be configured to extend across at least one of the holes of the base plate when the base and attachment plates are assembled together. The attachment plate may further include a flange extending from the attachment plate and defining a flange screw hole. The base and attachment plates may be attached by a screw extending through the elongated slot. The groove may be an elongated groove. An end portion of the base plate may be tapered. The attachment plate may include a plurality of grooves or notches disposed in series within the groove and may be configured to receive a rib extending from a top surface of the base plate. The elongated slot of the attachment plate may extend across a set of holes that includes the at least one hole of the holes when the base and attachment plates are assembled. The attachment plate may further include a head portion defining a longitudinal axis that is obliquely oriented with respect to a longitudinal axis of the elongated slot. The head portion may include a cluster of holes. A bottom surface of the head portion may be concave. The attachment plate may further include two arms extending therefrom. The two arms may extend from the attachment plate such that one of the two arms includes a bendable segment or a hook extension. The hook extension may include a first prong and a second prong in a dual pronged arrangement. One of the two arms may include a plurality of bendable segments. One of the two arms may include hooks extending therefrom. Some of the hooks may be suture hooks adapted to be attached to sutures. One of the suture hooks may be a dual pronged hook. All the suture hooks may extend from the same side of the one of the two arms. The base plate may include a cable groove and an oblong k-wire hole. A longitudinal axis of the cable groove may be perpendicular to a longitudinal axis of the oblong k-wire hole.

In yet another aspect, the present disclosure relates to a bone plate including a shaft portion and a head portion. The head portion may have a first bendable arm and a second bendable arm extending from the shaft portion. The first and second bendable arms may define a first set of holes and a second set of holes, respectively. The first and second set of arms may have a first suture hook and a second suture hook, respectively. The first and second suture hooks may extend toward a longitudinal axis of the bone plate. The bone plate may further include at least one of a first prong extending from a first terminal hole of the first set of holes and a second prong extending for a second terminal hole of the second set of holes. The first and second suture hooks may extend from adjacent the first and second terminal holes, respectively. The first suture hook and the first prong may be perpendicular to each other.

In accordance with another aspect of the present disclosure, a bone plate may be implanted by a process. In the process, a shaft portion of the bone plate may be attached to the bone along a longitudinal axis of the bone by inserting bone screws into screw holes defined by the shaft portion. And a head portion of the bone plate may be attached to the bone by bending arms of the head portion around contours of the bone. A prong for each of the arms may be inserted into the bone, and bone screws may be inserted into screw holes defined by the arms. Attaching the shaft portion may further include inserting K-wire through an oblong hole in the shaft portion. Attaching the head portion may further include securing suture to suture hooks extending from the arms of the head portion. The head portion may be attached to a trochanteric portion of the bone.

In accordance with yet another aspect of the present disclosure, a bone plate may be implanted by a process. In the process, a fixation plate may be attached to a bone along a longitudinal axis of the bone by inserting bone screws into screw holes defined by the fixation plate. And an attachment plate may be attached to the fixation plate by aligning and placing a grooved portion of the attachment plate over an end portion of the fixation plate and inserting an attachment screw through a first hole in the fixation plate and a second hole in the attachment plate. The attachment plate may be attached to the bone by inserting bone screws into screw holes defined by the attachment plate. The attachment of the attachment plate to the fixation plate may further include sliding the attachment plate along a longitudinal axis of the fixation plate to determine proper placement of the attachment plate. The attachment of the attachment plate to the bone may also include bending arms extending from the attachment plate around the bone. The process may further include bone screws that are inserted into screw holes defined by the arms of the attachment plate. The process may further include suture that is wrapped around suture hooks extending from an arm extending from the attachment plate.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIG. 1 is a top view of a bone plate in accordance with an aspect of the present disclosure;
FIGS. 2A-2G are perspective views of different portions of the proximal end of the bone plate of FIG. 1;
FIGS. 3A-3B illustrate schematic views of the bone plate of FIG. 1 implanted on a femur;
FIG. 4 is a top view of a bone plate in accordance with another aspect of the present disclosure;
FIG. 5 is schematic view of a bone plate system in accordance with another aspect of the present disclosure;
FIG. 6 is an enlarged bottom view of a portion of the bone plate system of FIG. 5;
FIG. 7 is an enlarged top view of a portion the bone plate system of FIG. 5;
FIG. 8 is an enlarged top view of a portion of the bone plate system of FIG. 5;
FIG. 9 is an enlarged side view of a portion of the bone plate system of FIG. 5;
FIG. 10 is a top view of a bone plate of the bone plate system of FIG. 5;
FIG. 11 is an enlarged bottom view of a portion of the bone plate of FIG. 10;
FIG. 12 is an enlarged top view a portion of the bone plate of the bone plate system of FIG. 5.
FIG. 13 is a top view of a bone plate in accordance with another aspect of the present disclosure;
FIGS. 14-17 illustrate various bone plates in accordance with another aspect of the present disclosure;
FIG. 18a is a side view of an instrument in accordance with an aspect of the present disclosure;
FIG. 18b is a close-up view of the instrument of FIG. 18a;
FIG. 19 is a cross-sectional view of the instrument of FIG. 18a engaged with a bone plate in accordance with an aspect of the present disclosure;
FIG. 20 is a perspective view of a bone fixation system including a bone plate and instruments in accordance with an aspect of the present disclosure;
FIG. 21 is a cross-sectional view of an aspect of the bone fixation system of FIG. 20 taken along Q-Q';
FIG. 22 is a side view of one of the instruments in FIG. 20;
FIG. 23 is a side view of a distal portion of the instrument of FIG. 22;
FIG. 24 is a side view of one of the instruments in FIG. 20
FIG. 25 is a side view of a distal portion of the instrument of FIG. 24;
FIG. 26 is a top view of an attachment plate in accordance with an aspect of the present disclosure;
FIG. 27 is a top view of a bone plate in accordance with an aspect of the present disclosure; and
FIG. 28 is a top view of the bone plate of FIG. 27 attached to an attachment plate in accordance with an aspect of the present disclosure.

### DETAILED DESCRIPTION

As used herein, the term "proximal," when used in connection with a surgical tool or device, or components of a device, refers to the end of the device closer to the user of the device when the device is being used as intended. On the other hand, the term "distal," when used in connection with a surgical tool or device, or components of a device, refers to the end of the device farther away from the user when the device is being used as intended. However, when used in connection with the human body, the term "proximal" means closer to the heart, and the term "distal" means further from the heart. As used herein, the terms "substantially," "generally," "approximately," and "about" are intended to mean that slight deviations from absolute are included within the scope of the term so modified, such as deviations of up to 10% greater or lesser than absolute. Such directional terms are not intended to suggest that any features of the devices described herein must exist in any particular orientation when constructed. To aid the Patent Office and any readers of any patent issued on this application in interpreting the claims appended hereto, Applicant notes that it does not intend any of the appended claims or claim elements to invoke 35 U.S.C. 112(f) unless the words "means for" or "step for" are explicitly used in the particular claim.

The present disclosure includes various embodiments of bone plates configured to hold parts of a fractured bone, such as a femur, together to promote healing of the fractured parts. The various embodiments described herein will be discussed in the context of fixing a fractured femur. However, it should be noted that these embodiments may be used in other ways including, but not limited to, fixing other fractured bones such as the tibia, the humerus, etc. Throughout the description, the same reference numerals are used to refer to identical or similar elements in different embodiments, but within a different series of numbers (*e.g*., 100-series, 200-series, 300-series, etc.).

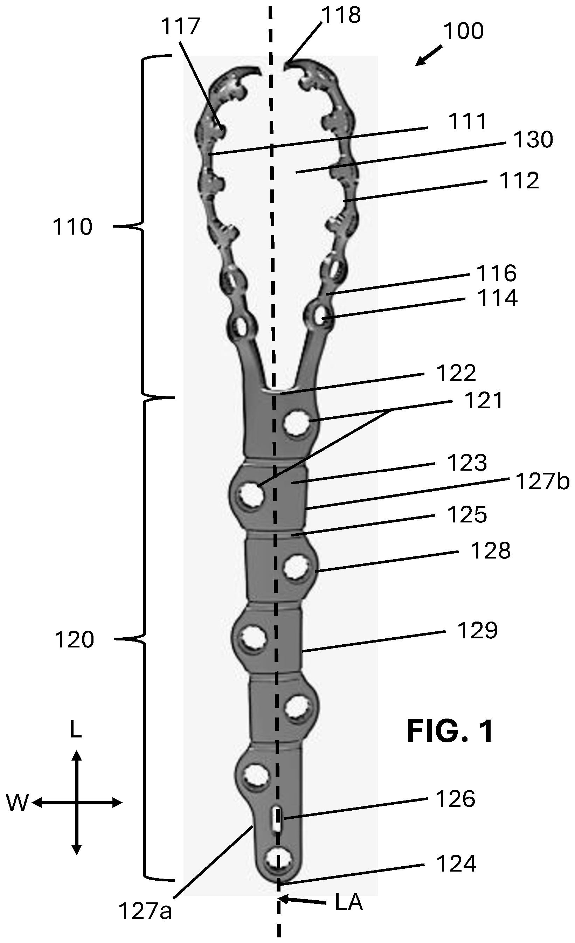

FIGs. 1-3B show a bone plate system 100 having head portion 110 and a shaft portion 120. The head portion extends from a first end 122 of the shaft portion opposite of a second end 124 of the shaft portion. Shaft portion 120 defines a top surface 123, a bottom surface (not shown) opposite of the top surface, a first side portion 127a and a second side portion 127b between the top and bottom surfaces. Shaft portion 120 also includes screw holes 121 extending through top surface 123 and the bottom surface. Top surface 123 has cable grooves 125 that extend across a width (W) of shaft portion 120 and are disposed between screw holes 121. Cable grooves 125 are configured to receive wires and/or cables of various sizes (e.g., vitallium cables from Dall-Miles Cable System and the like ranging from 1-3 mm in diameter). First and second side portions 127a,b include planar segments 129 and curved segments 128 that extend laterally away from the longitudinal axis (LA) of shaft portion 120. Planar segments 129 and curved segments 128 are alternatively disposed along first and second side portions 127a,b of shaft portion 120 and separated by cable grooves 125. Some of screw holes 121 are positioned partially within curved segments 128 such that an outer extent of the screw holes are positioned further from the longitudinal axis (LA) of shaft portion 120 than planar segments 129.

Screw holes 121 are adapted to receive both locking and non-locking screws and may include a polyaxial locking feature within the inner circumference of the hole to allow such screws to be arranged in polyaxial orientations. For example, screw holes 121 may be adapted to allow a screw to move within a range between about 15 and 45 degrees from a central axis of a respective screw hole. Screw holes 121 may be threaded or unthreaded and may have a countersink extending from the top surface 123 toward the bottom or bone contacting surface of the shaft portion 120. It is contemplated that the locking features of the holes may be any type of design, including, but not limited to, threaded holes or holes including SMARTLock^{®} technology as offered by Stryker Corporation. In some embodiments, the screws holes disclosed herein may be the same or similar to the screw holes described in U.S. Patent Nos. 11,039,865, 11,291,484, and/or 8,574,268, the disclosures of which are hereby incorporated by reference in their entirety. In some instances, the screw holes 121, 114 may have a universal design configured to be engaged by locking screws, non-locking screws, cable plugs for cable systems (e.g., Dall-Miles Cable System), dowels designed to link nail and plate (e.g., Alpha^{™} Dowel from Stryker), and various instruments including plate inserters, drill guides (e.g., ball-and-cone variable angle drill guides), benders, and bender tips (discussed in more detail below). In this manner, the bone plates having such universal screw holes afford medical personnel a variety of options regarding implant arrangements and implantation methods.

The bone plates disclosed herein may also be configured to link to bone nails and other implants as described in U.S. Patent No. 12,144,529, the disclosure of which is hereby incorporated by reference in its entirety. Additionally, the bone plates may be configured to attach to a guide block and/or a plate inserter assembly including a handle (e.g., Delta handle) for inserting/targeting as described in U.S. Patent Application Publications Nos. 2025/0169861 and 2024/0156499, respectively, the disclosures of which are hereby incorporated by reference in their entirety.

Shaft portion 120 further comprises an oblong hole 126 positioned proximate to second end 124 of the shaft portion between two of the screw holes 121 that is adapted to receive K-wire, olive K-wire(s) or the like and allow the wire to slide to facilitate compression of a bone fracture. Oblong hole 126 extends along the length (L) of shaft portion 120 and is oriented perpendicular to cable grooves 125. The bottom surface of shaft portion 120 may define a curved shape adapted to correspond or conform to a curved shape of a patient's bone such a femur. The shaft portion 120 and head portion 110 may be made from the same or different materials. In some instances, shaft portion 120 and head portion 110 may be formed of Titanium Grade 5 (Ti 6AI-4V) or another biocompatible metal such as stainless steel. Alternatively, head portion 110 may be made from a less stiff material to allow for increased flexibility, e.g., Titanium Grade 2 or other metals similar thereto.

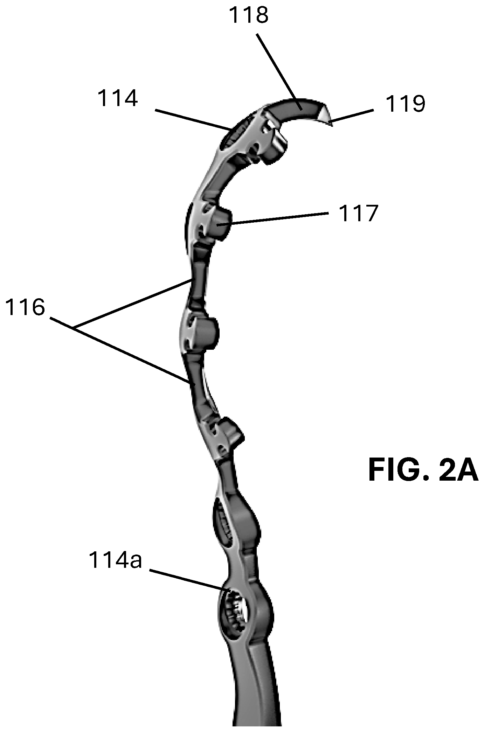

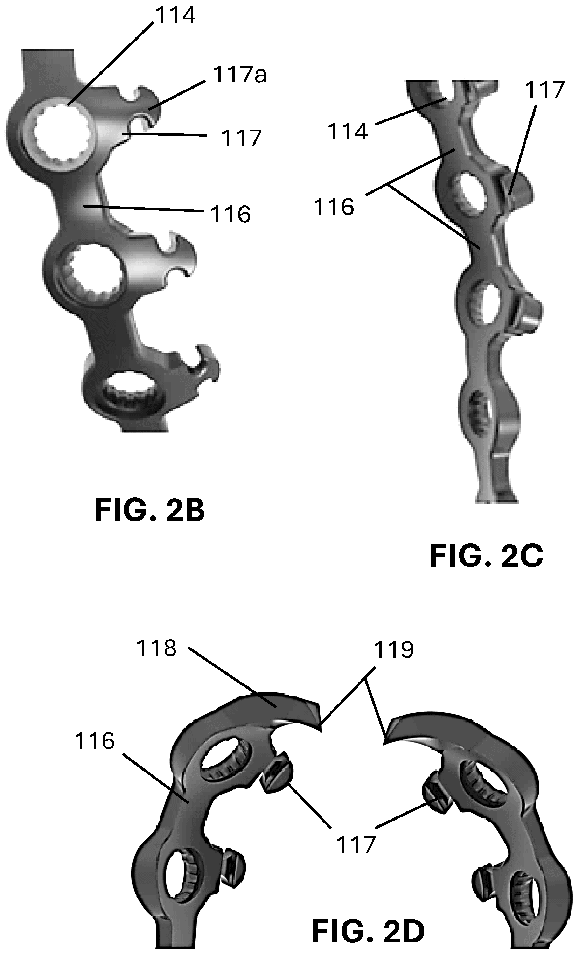

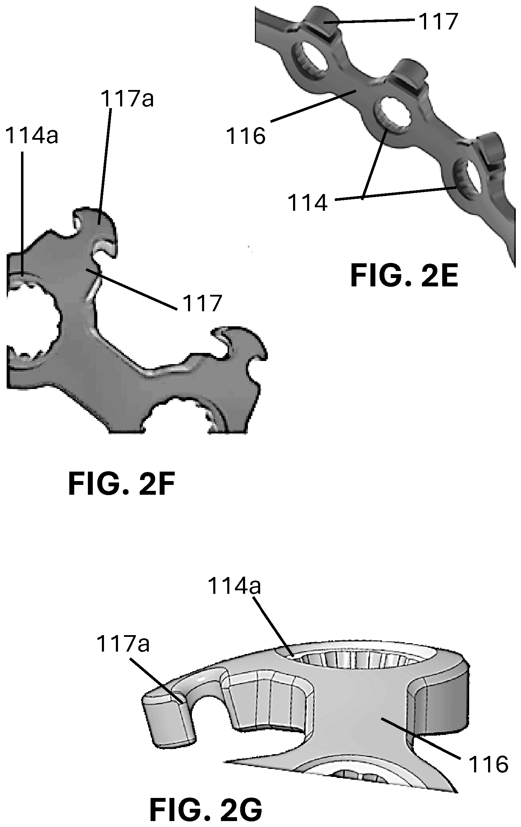

Head portion 110 of bone plate system 100 includes a first appendage or first arm 111 and a second appendage or second arm 112 connected to and extending away from shaft portion 120. First and second arms 111, 112 create a substantially U-shape to define an open region 130 as the arms extend from shaft portion 120. In other instances, the first and second arms may create a substantially V-shape to define an open region therebetween. Both first and second arms 111, 112 include bendable sections 116, screw holes 114 disposed between the bendable sections, suture hooks 117 and prongs 118. Screw holes 114 are similar to screw holes 121 described above with respect to their engagement with bone screws and adapted to receive locking and non-locking bone screws to secure first and second arms 111, 112 to a patient's bone. In some instances, the number of screw holes 114 in both arms 111, 112 exceeds the number of screw holes in the shaft portion 120. Screw holes 121 and 114 include a recessed portion 114a, as best shown in FIG. 2G. In many instances, this recessed portion 114a is a countersink or some cutout of the screw hole configured for, among other things, engagement with a bending instrument. Screw holes 114 may be threaded or unthreaded and may have different sizes and shapes to accommodate various of bone screws.

Each suture hook 117 extends from adjacent one of the screw holes 116 toward the other of the first or second arms 111, 112 and includes a dual pronged hook 117a (best shown in FIG. 2B). Suture hooks 117 are adapted to receive and attach to suture (e.g., absorbable or non-absorbable sutures #2 to #5) or wire lines (not shown) that may be used to secure first and second arms 111, 112 to a patient's bone. It is contemplated that, in other embodiments, suture hooks 117 may extend in other directions not shown or discussed herein. Prongs 118 each define a tine configured to be impacted into bone. For example, the prongs/tines 118 may be impacted into bone with an instrument (e.g., a hammer or impactor) such that the prongs establish an impaction fit within the bone. In some instances, suture hooks 17 and prongs 118 may be oriented perpendicular to each other. Prongs 118 extend from adjacent a terminal hole 114a of screw holes 114 and taper to a sharp point 119 that is adapted to cut through bone tissue to secure first and second arms 111, 112 to a patient's bone. In this manner, first and second arms 111, 112 of bone plate 100 can be fixed to a patient's bone with various mechanisms including, but not limited to, bone screws corresponding to screw holes 114, suture or wire attached to suture hooks 117, prongs 118 extending bone, and any combination thereof. For example, wires may be wrapped around the first and second arms and the bone such that the wires extending across bendable sections 116. In such instances, bendable sections 116 may include grooves or valley-like features configured to receive wire extending across the bendable sections 116. In this and other instances, the prongs 118 may be configured to initially fix first and second arms 111, 112 to a patient's bone before other fixation mechanisms (e.g., bone screws, K-wires, sutures, etc.) are deployed to further secure the arms to the bone.

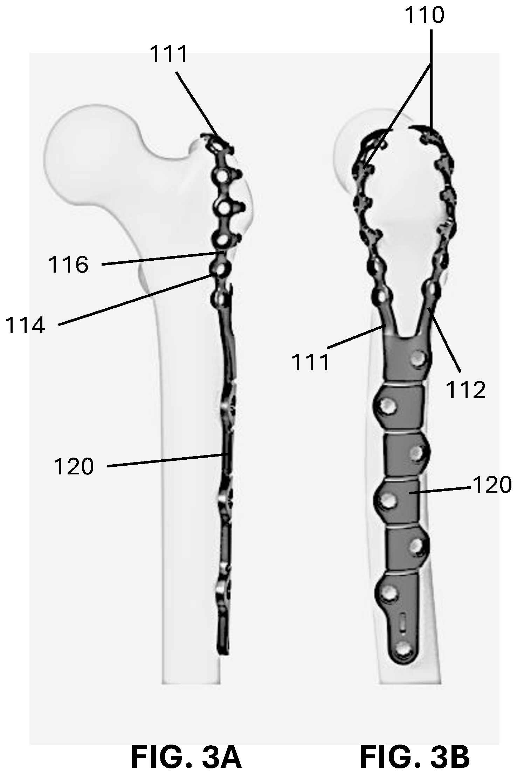

Bendable sections 116 of first and second arms 111, 112 define a width that is smaller than the outer diameter of screw holes 114 but larger that the inner diameter of the screw holes. Bendable sections 116 are adapted to bend such that they can conform to the curved features of a patient's bone and are made from a biocompatible material that is malleable or pliable but still has enough structural rigidity to hold fractured bone parts together. As noted above, such material may include, but not limited to, Titanium Grade 5. Alternatively or in addition to, the bendability or flexibility of first and second arms 111, 112 may also be related to the dimensions (e.g., length, width, and height or thickness) of bendable sections 116. For example, some sections of the arms may be thinner or narrower than other sections such that the flexibility or bendability various across the different sections of the arms. In such instances, bendable sections 116 have a smaller second moment of inertia (i.e., area moment of inertia) than screw holes 114, which causes bendable sections 116 to bend before screw holes 114. In other examples, the bendable sections may have substantially uniform dimensions that allow them to bend and flex while still providing enough rigidity to hold fractured bone parts together. In some instances, the bendability or flexibility of first and second arms 111, 112 may also be related to a weakened section of the arms. Bendable sections 116 are more pliable or malleable than shaft portion 120 and, in some cases, significantly more so depending on the dimensions and materials used. This allows first and second arms 111, 112 to bend or twist away from each other to conform to the contours of bone, such as the contours of a trochanteric region of a femur, and thereby improve fixation to bone. For example, first and second arms may be curved away from and then back toward the longitudinal axis (LA) of the plate in a hook or claw shape, which allows the arms to conform and wrap around a trochanteric region of a patient's femur (as shown in FIGs. 3A-B). Because bendable sections 116 are disposed between screw holes 114, the screw holes of first and second arms 111, 112 may be individually articulated or adjusted so that they may be placed tightly against curved or rounded portions of a patient's bone, as shown in FIGs. 3A and 3B. In this manner, first and second arms 111, 112 of bone plate 100 are adapted to conform to and tightly wrap around curved or rounded, conventional and unconventional, bone structures to hold fractured bone parts together.

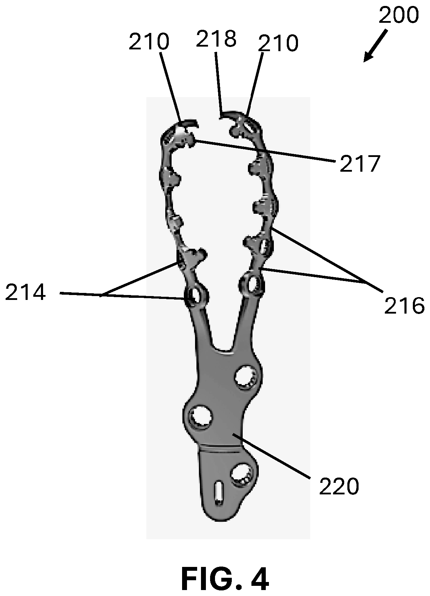

FIG. 4 shows a bone plate system 200 having head portion 210 and a shaft portion 220, according to another aspect of the present disclosure. Head portion 210 and shaft portion 220 the same or similar features as described above for bone plate 100. However, shaft portion 220 is shorter than that of shaft portion 120. Shaft portion 120 may have a length ranging from about 150 mm to 170 mm, more preferably 155 mm to 157mm, and shaft portion 220 may have a length of about 50 mm to 80 mm, more preferably 65 mm to 68 mm.. The total length of the bone plates described herein range from about 150 mm to 260 mm in length. For example, there may be two sizes of trochanter plates, and each size may come in two different shaft lengths. A large size may have six or more screw holes on each arm and a shaft having either three or seven screw holes. In some instances, an overall length of a large plate having seven screw holes in the shaft may be about 253 mm in length, and a plate having three screw holes in the shaft may be about 164 mm long. As for a small trochanter plate, it may have five screw holes in each arm and may have a shaft having either three or seven holes. The overall length of a small plate having seven holes in the shaft may be about 241 mm in length, or it may be about 152 mm long when the small plate only has three holes in the shaft. The length difference between the large and small plates may be associated with the five or six screw holes in each of the arms. A shaft having seven screw holes may be about 156 mm long. A shaft having three screw holes may be about 67 mm long. In this manner, physicians have the option of selecting a bone plate having a smaller shaft that may be better suited for treatment and may reduce time and resources needed for implantation.

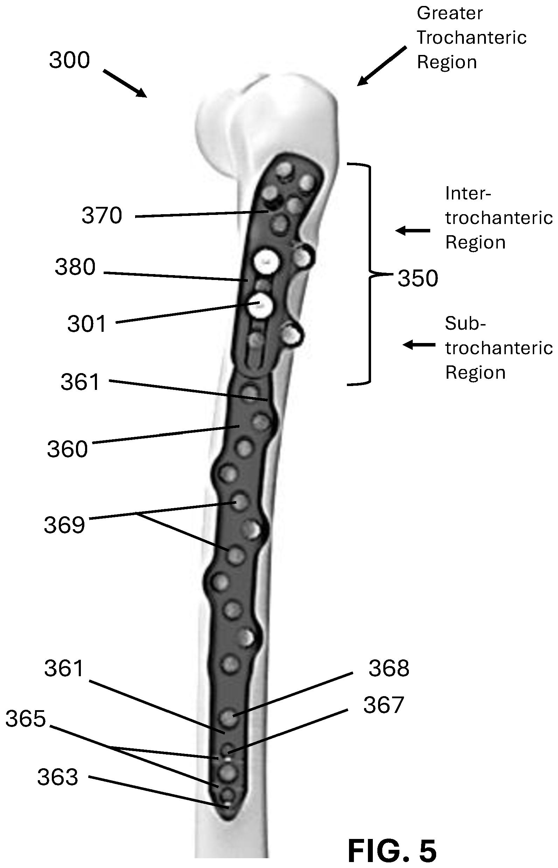

FIGS. 5-12 depict a bone plate system 300 according to another aspect of the present disclosure. While bone plates 100 and 200 are of an integral construction having head portion 110, 210 and shaft portion 120, 220, bone plate system 300 includes an attachment plate 350 and a fixation plate 360 that are two separate pieces or parts connectable to one another to form bone plate 300. In the illustrated embodiment, attachment plate 350 is attached to fixation plate 360 via connecting screws 301, although in other embodiments the plates may be connected by any means or mechanism known in the art.

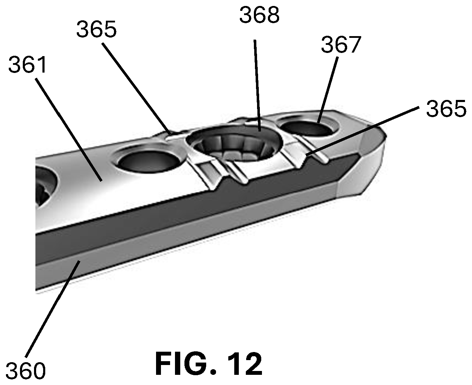

Fixation plate 360, 360a includes an attachment portion 361, 361a having a set of holes 362 and a tapered end 363 at one or both ends of the fixation plate. In some instances, the fixation plate has a symmetrical design to allow for attachment plate 350, 350a to be attached to either end thereof. The fixation plate 360 may be used for left and right femur bones. FIG. 5 shows fixation plate attached a left femur bone. For a right femur bone, fixation plate 360 may be turned 180° to match the curvature of the right femur, and attachment plate 350 will again be placed proximally thereto. In some instances, other attachments, e.g., for the distal femur, may be attached to the distal end of the fixation plate 360. In such instances, these distal end attachments may be attached to the fixation plate in the same or similar manner as the attachment plate 350. The set of screw holes 362 includes connecting screw holes 367 and bone screw holes 368 such that attachment portion 361 is configured to be attached to both a patient's bone using the bone screw holes and another plate (*e.g*., attachment plate 350 via connecting screw holes 368) using the connecting screw holes. Generally, connecting screw holes 367 and bone screw holes 368 are aligned in a linear pattern, but other non-illustrated embodiments may include various hole patterns. Attachment portion 361 also includes multiple ribs or ridges 365 that extend from a top surface of fixation plate 360. Each of the multiple ridges may have a trapezoidal prism-like shape, as best shown in FIG. 12, or a rounded shape. The multiple ribs or ridges 365 allow fixation plate 360 the ability to interlock or engage another plate (*e.g*., attachment plate 350) defining corresponding features design to connect or engage with the ribs or ridges, as discussed below.

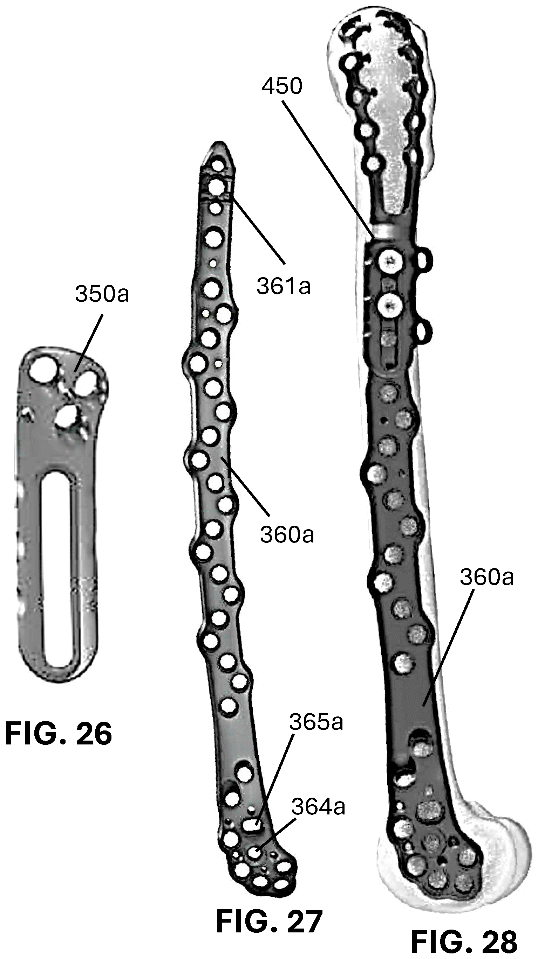

Fixation plate further includes screw holes 369 disposed in a zig-zag line pattern extending along the length of the fixation plate 360. Screw holes 369 are the same or similar to the screw holes 121 described above. For example, screw holes 369 may include locking features of any type of design, including, but not limited to, threaded holes or holes including SMARTLock^{®} technology as offered by Stryker Corporation. In some instances, the fixation plate may include a monoaxial screw hole 364a at one end of the plate, as shown in FIG. 27. The monoaxial screw hole 364a may be located adjacent an oblong hole 365a. In such instances, the monoaxial screw hole 364a and oblong hole 365a may be located in an enlarged region of the fixation plate located at an end of the plate furthest from or opposite to the tapered end designed to attach to an attachment plate, as shown in FIG. 28.

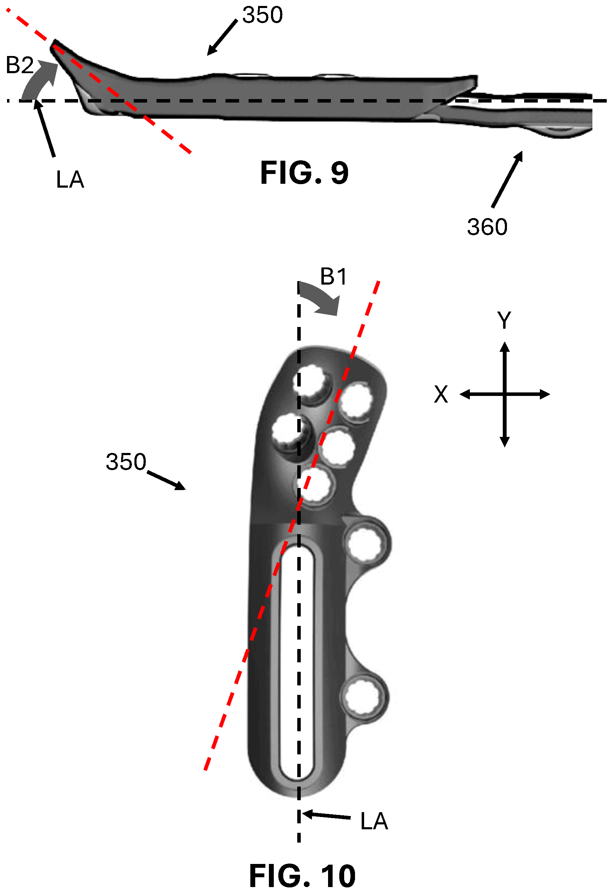

Attachment plate 350 comprises a head portion 370 and a shaft portion 380, as shown in FIGS. 5-10. Head portion 370 includes screw holes 374 clustered together and is obliquely oriented with respect to the longitudinal axis (LA) of shaft portion 380 within an X-Y plane, as shown in FIG. 10. Additionally, head portion 370 is bent up or has an upward trajectory such that it is also obliquely oriented with respect to the longitudinal axis (LA) of shaft portion 380 in a Y-Z plane, as shown in FIG. 9. In other words, as illustrated in FIG. 10, a top surface 371 of head portion 370 simultaneously defines a first bend (B1) in a first plane (e.g., X-Y plane) and a second bend (B2) in a second plane (e.g., Y-Z plane) resulting in a combination bend having a slanted upward trajectory. These bends defined by head portion 370 allows attachment plate 350 to conform to the intertrochanteric region of a femur where the bone curves upward from the subtrochanteric region to the greater trochanteric region, as shown in FIG. 5. Generally, head portion 320 has a stiffness similar to that of shaft portion 310. Head portion also includes a variable thickness between top surface 371 and bottom surface 372 such that some of the screw holes 374 have a greater depth than the others, as shown in FIG. 10.

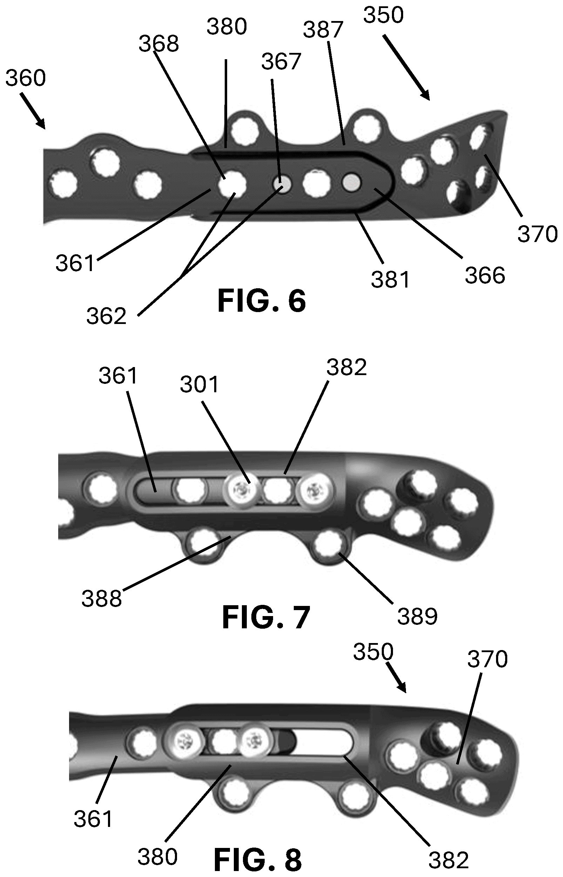

Shaft portion 380 includes a groove 381 shaped to correspond to the profile and dimensions of attachment portion 361 of fixation plate 360 and having an elongated slot 382. For example, groove 381 (*i.e*., a receiving portion) that may be adapted to receive attachment portion 361 such that a bottom surface 366 of fixation plate 360 is flush or level with a bone contacting surface 387 of shaft portion 380, as best shown in FIG. 6. Alternatively, groove 381 may receive attachment portion 361 such that bottom surface 366 of fixation plate 360 extends slightly beyond bone contacting surface 387 of shaft portion 380.

Elongated slot 382 extends along the length of groove 381 of shaft portion 380 and is configured to align with the set of holes 362 of attachment portion 361 when the attachment plate is positioned within groove 380 of shaft portion 380. In some arrangements, the entire attachment portion 361 may be placed within groove 381 such that the set of holes 362 are positioned proximate to head portion 370, as shown in FIG. 7. Alternatively, attachment portion 361 may be partially placed within groove 381 such that the set of holes 362 are spaced apart from head portion 370, as shown in FIG. 8. In this manner, attachment plate 350 and fixation plate 360 may be attached in various configurations to allow physician's the ability to adjust an overall length of bone plate system 300 to best treat a patient's condition. Shaft portion 380 further comprises two flanges 388 extending from laterally away from the longitudinal axis of shaft portion 380. Each flange 388 defines a screw hole 389 that is similar to the other screw holes described herein.

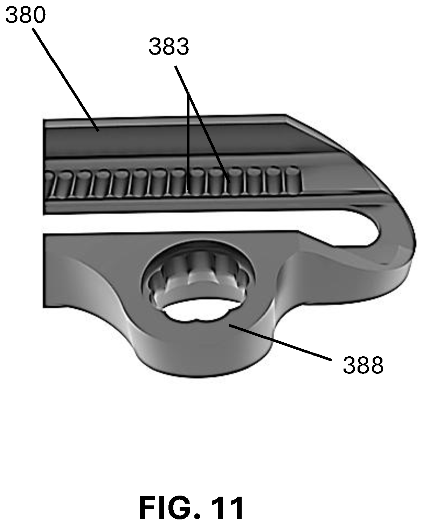

Additionally, groove 381 includes notches 383 disposed in series along one or both sides of elongated slot 382 in a corrugated or a washboard-like formation, as shown in FIG. 11. Each of the notches 383 is adapted to receive a respective ridge of the multiple ridges 365 extending from fixation plate 360, shown in FIG. 12. In particular, the size and shape and spacing between the notches 383 correspond to that of ridges 365 so that the ridges and notches are adapted to be mated together when attachment plate 350 is connected to 360 fixation plate with connecting screws 301. In this manner, ridges 365 and notches 383 allow fixation plate 360 and attachment plate 350 to be coupled together in this interlocking arrangement so that bone plate system 300 can provide structural support for fractured bone parts.

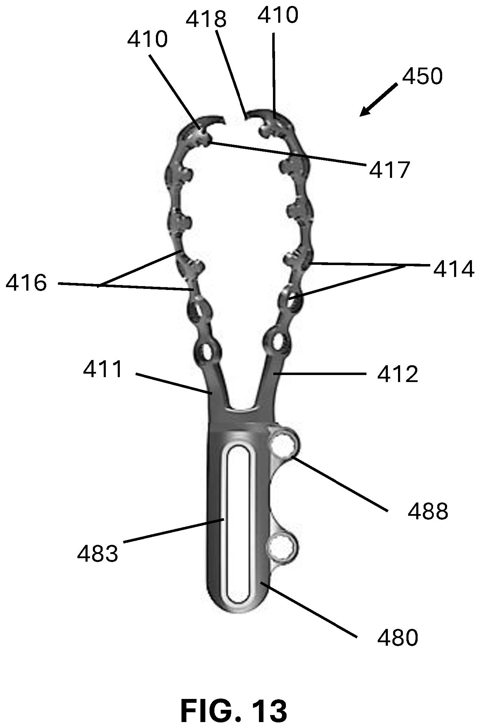

FIG. 13 illustrates another embodiment of attachment plate 450 having head portion 410 and shaft portion 480. Shaft portion 480 is similar to that of shaft portion 380 described above such that it includes a groove 481, an elongated slot 482, notches 483, flanges 488, etc. Additionally, shaft portion 480 is adapted to connect to attachment portion 361 of fixation plate 360 in the same or similar manner shaft portion 380. Head portion 410 is similar to head portion 110 such that head portion 410 includes a first arm 411 and second arm 412 that have bendable sections 416, screw holes 414, suture hooks 417, and prongs 418 which are the same or similar to those described above. Head portion 410 is adapted to wrap around and conform to bone in the same or similar manner as described above for head portion 110.

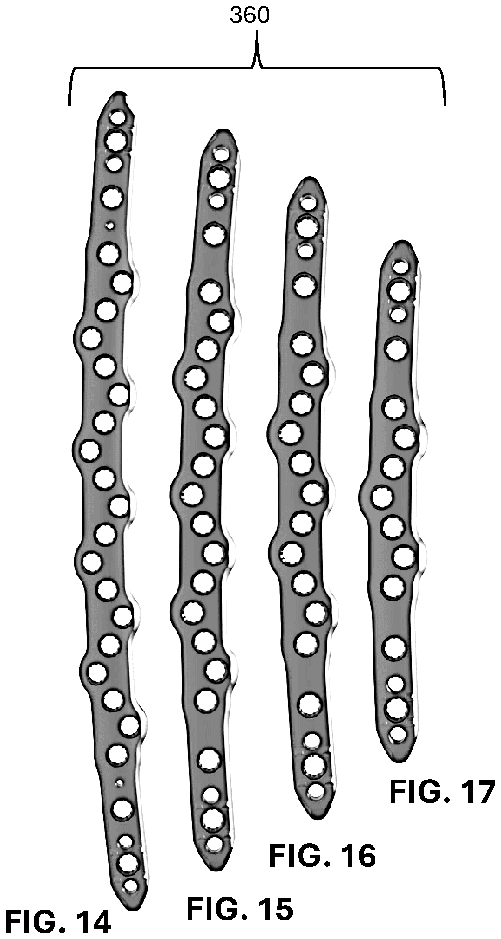

As depicted in FIGS. 14-17 and 27-28, fixation plates 360, 360a and attachment plates 350, 350a, 450 may come in a variety of different sizes and configurations. While screw holes patterns for the different sizes may vary, the attachment portions at the ends of the fixation plates have the same or similar hole pattern so that any one of the attachment plates can attach each of the fixation plates regardless of size. In other words, the fixation plates have universal attachment portion disposed at least one end of the plate.

In some instances, the bone plates described herein may be included in a kit having a various plates and attachments. For example, a kit may include one or more bone plates, all having an attachment portion (*e.g*., attachment portion 361) at one end of the plate, and a variety of attachment plates having different head portions and the same or similar shaft portions such that all the attachments plates are adapted to attached to the attachment portion of the bone plates.

A method of assembling an attachment plate (e.g., attachment plate 350, 450) with another bone plate (e.g., fixation plate 360, 360a) will now be described. First, a fixation plate may be fixed to a patient's bone. Then an attachment plate may be placed over a fixation plate such that a groove (e.g., 381) of the attachment plate is aligned with and mated to an attachment portion (e.g., attachment portion 361) of the fixation plate. Alternatively, an attachment portion (*e.g*., attachment portion 361) of a bone plate may be inserted into a groove (*e.g.*, groove 381) of an attachment plate that is configured to receive the attachment portion prior to implantation of the plates. In either case, ridges (*e.g*., 365) extending the attachment portion are aligned with notches (*e.g*., notches 383) located within the groove of the attachment plate such that the bone contacting surfaces of the attachment plate and the bone plate are level or flush with each other. Finally, a pair of connecting screws (*e.g*., connecting screws 301) are inserted through an elongated slot (*e.g*., elongated slot 382), defined by the groove of the attachment plate, and then threaded into screws holes disposed in the attachment portion of the bone plate. This method may include removing the connecting screws and sliding the attachment portion out of or into the groove of the attachment plate to adjust the overall length of the bone plate assembly.

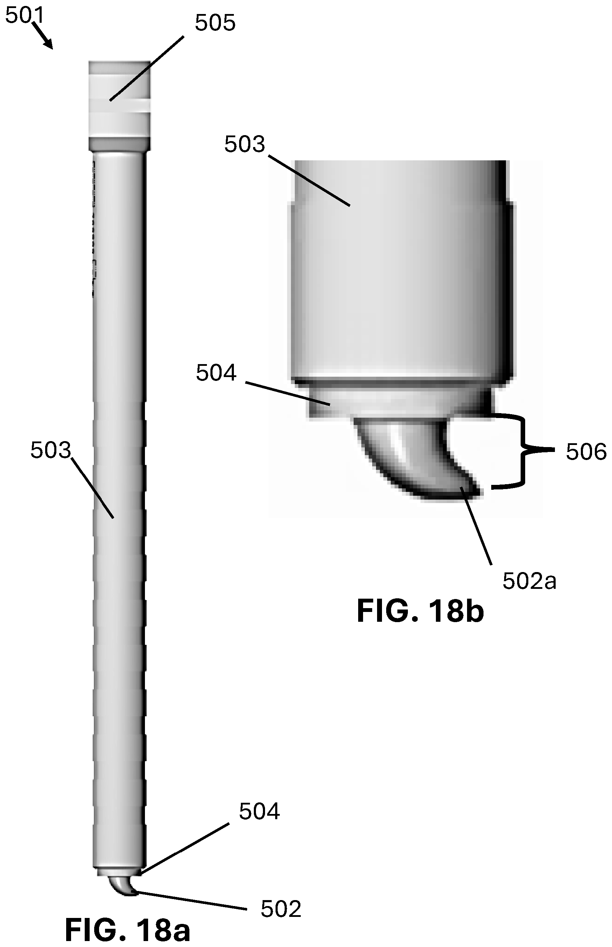

Another aspect of the present disclosure relates to an instrument or instruments designed to bend various bone plates including those described herein. For example, as shown in FIGS. 18a and 18b, a bending instrument 501 includes a hook 502 and a shoulder 504 disposed adjacent to the hook. The hook 502 extends from the shoulder 504, and the shoulder 504 extends from a shaft 503 of the bending instrument 501 that is generally cylindrical in shape or at least includes a cylindrical portion, but not necessarily limited thereto. The shaft 503 has a larger outer diameter than the shoulder 504 such that a stepped surface is located where the bending instrument 501 transitions from the shaft 503 to the shoulder 504. The hook 502 is tapered to a pointed tip 502a and curved to form a gap 506 between and an end of the shoulder 504 and the pointed tip 502a. The gap 506 is configured to receive a portion of a bone plate such that the shoulder 504 and the pointed tip 502a can be positioned on opposite sides of the plate (e.g., a bone contacting side and a non-bone contacting side). The distance between the pointed tip 502a and the shoulder 504 (i.e., gap 506) is fixed. As such, the bending instrument may be offered in a variety of shapes and sizes to accommodate the dimensions and structural characteristics of various bone plates.

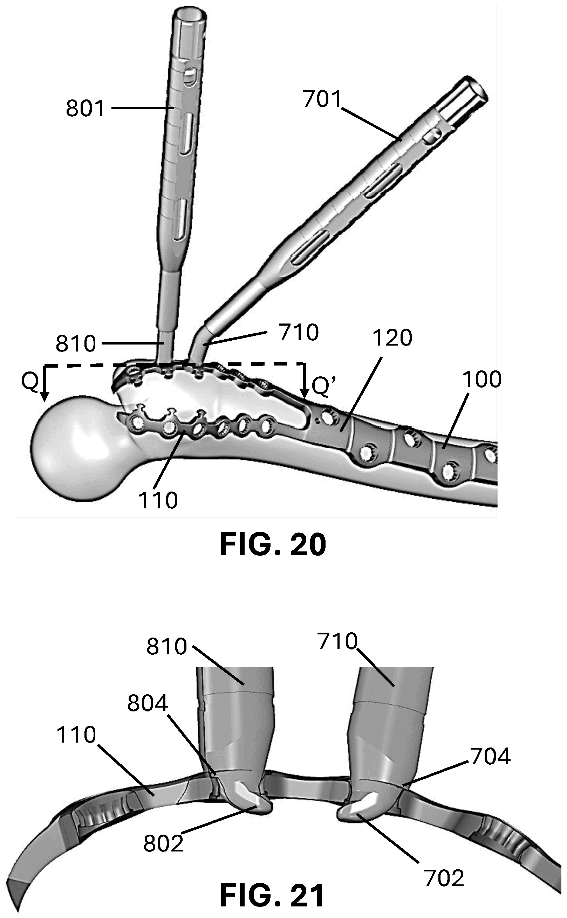

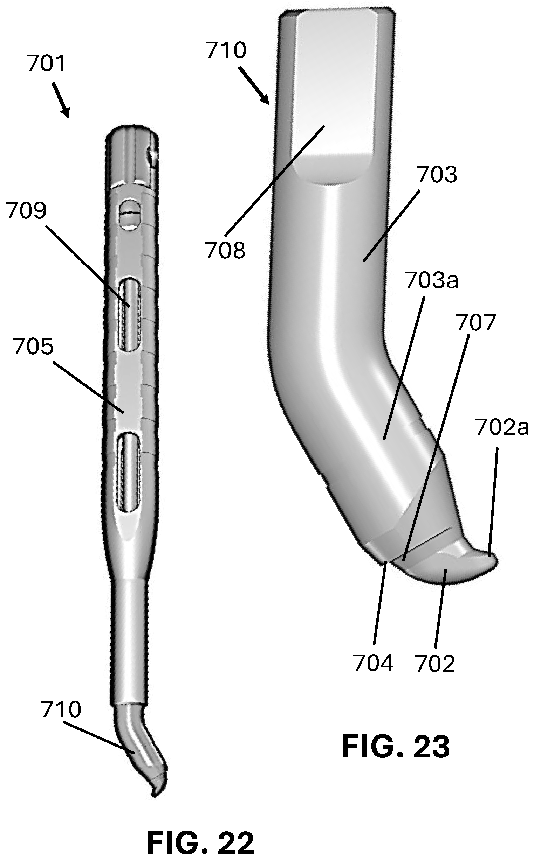

An additional aspect of the present disclosure relates to a bone fixation system that includes a bone plate or plates and instruments having a modular design adapted to bend various bone plates including those described herein. For example, as shown in FIGS. 20-25, a first bending instrument 701 and a second bending instrument 801 are configured to engage a bone plate (e.g., bone plate 100, 200, 360, 360a, 450) in a manner that such instruments may be used to bend the bone plate to conform to the contours of a patient's bone. First and second bending instruments 701, 801 each have a detachable tip 710, 810 that is threadably connected to internal attachment rod 709, 809. The detachable tips 710, 810 that have similar features as those described above for bending instrument 501, including a hook 702, 802 and a shoulder 704, 804 located near the hook such that they are configured to engage the screw holes (e.g., screw shoes 121, 114) in a bone plate.

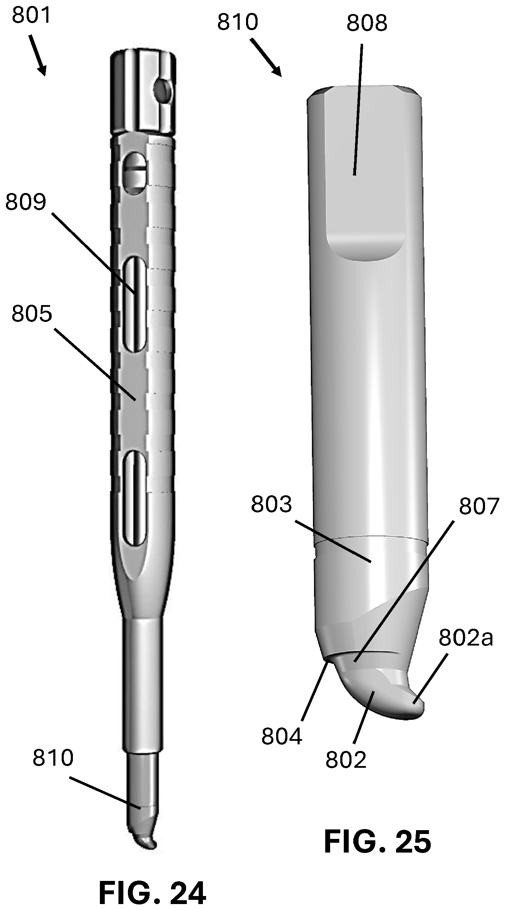

The hook 702, 704 extends from the shoulder 704, 804, and the shoulder extends from a shaft 703, 803 of the bending instruments 701, 801. The shaft 703, 803 is generally cylindrical in shape or at least includes a cylindrical portion. The detachable tip 710 of the first bending instrument 701 includes a bent portion 703a. Each shaft 703, 803 has an abrupt change in its outer diameter at the shoulder 704, 804 such that an undercut surface 707, 807 is located between the shoulder 704, 804 and the hook 702, 802. In some instances, the undercut surface 707, 807 extends from a side of the instrument that is facing in a direction opposite of the one in which the hook 702, 802 is extending (as best shown in FIGS. 24 and 25). Also, in some instances, the undercut surface 707, 807 does not extend entirely around the circumference of the instrument. The hook 702, 802 is tapered to a pointed tip and curved away from the shoulder 704, 804. In other words, the hook 702, 802 and shoulder 704, 804 extend in opposite directions from each other to form a proximal facing surface (on hook 702, 802) and a distal facing surface (on shoulder 704, 804). Additionally, the detachable tip 710, 810 for each of the first and second bending instruments 701, 801 includes an attachment end 708, 808 that is sized and shaped to fit into and attach to the handle 705, 805 of the instrument. In some instances, the handle 705, 805 has a modular design such that it is compatible with a variety of bender tips. For example, it is contemplated that various tips of different sizes or features may be compatible with the handle 705, 805 of the bend instrument such that a tip could be removed and replaced with another tip having a different size and/or features. In this manner, the bend instruments may be used to bend a variety of bone plates by simply switching the tip of the instrument for one that fits and engages with a screw hole of any given plate. Additionally, the bend instruments may be used to twist, pull, flex, curl, or otherwise adjust various aspects of such bone plates to include various bends and/or bend formations.

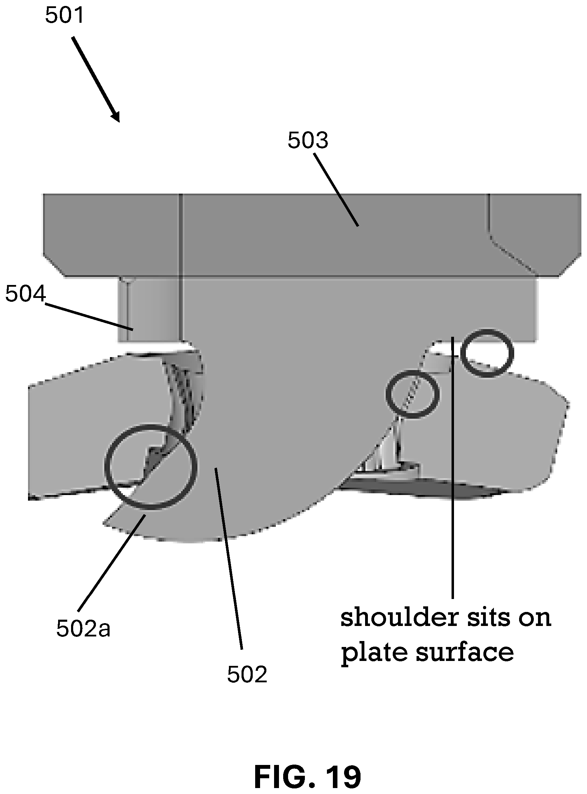

In yet another aspect, the present disclosure relates to methods for bending bone plates by using one or more bending instruments. For example, a bending instrument (e.g., bending instrument 501, 701, 801) having a shoulder and a hook extending from the shaft of the instrument (e.g., shoulder 504, 704, 804 and hook 502, 702, 802) may be used to engage and bend a bone plate. In such instances, the hook may be inserted into a screw hole such that a tip (e.g., pointed tip 502a, 702a, 802a) of the hook is placed underneath a lower surface (i.e., a bone contacting surface) of the bone plate, and the shoulder may then be positioned within a recessed portion (e.g., recessed portion 114a of screw hole 114) of the bone plate or above the recessed portion and against a top surface of the bone plate, as shown in FIGS. 19 and 21. The outer diameter of the shoulder may be larger or smaller than the inner diameter of the recessed portion (i.e., countersink of screw hole) so that the shoulder may be positioned above or within the recessed portion. If the bone plate has already been placed against the bone, the tip of the hook would be positioned between the bone and the bone contacting surface of the plate. Once the bone plate is engaged by the bending instrument, a shaft of the bending instrument may be maneuvered in a joystick-like manner to bend the bone plate. In some instances, two or more of these joystick-like tools (i.e., bending instruments) may be used by surgeons to bend bone plates, including bendable arms extending from the bone plates, to conform to the anatomy of a patient. In this manner, the bending instruments described herein may be used to bend bone plates that do not have threaded holes or features that facilitate a threaded engagement with such instruments.

It is contemplated that the some of the bone plates and instruments may be included in a kit. For example, one or more bending instruments 501, 701, 801 may be included in a kit along with various bone plates (e.g., plate 100, 200, 300, 400).

It is to be understood that the disclosure set forth herein includes any possible combinations of the particular features set forth above, whether specifically disclosed herein or not. For example, where a particular feature is disclosed in the context of a particular aspect, embodiment, arrangement, or configuration, that feature may also be used to the extent possible, in combination with and/or in the context of other particular aspects, embodiments, arrangements, and configurations of the technology, and in the technology in general. Furthermore, although the technology here has been described with reference to particular features and figures, it is to be understood that these features are merely illustrative of the principles and applications of the present technology. It is therefore to be understood that numerous modifications, including changes in the sizes of the various features described herein, may be made to the illustrative arrangement and that other arrangements may be devised without departing from the spirit and scope of the present technology. In this regard, the present technology encompasses numerous additional features in addition to those specific features set forth in the claims below. Moreover, the foregoing disclosure should be taken by way of illustration rather than by way of limitation as the present technology is defined by the claims set forth below.

## Claims

1. A femur plate, comprising:
a shaft portion defining a plurality of holes; and
a head portion extending from an end of the shaft portion and having two arms configured to at least partially wrap around and attach to trochanters of a femur, each of the two arms defining a plurality of holes and at least one of the two arms has a suture hook extending from an adjacent one of the plurality of holes.

2. The femur plate of claim 1, wherein the shaft portion and the head portion are separate pieces attached to each other.

3. The femur plate of claim 2, wherein the head portion includes a receiving portion configured to receive an attachment portion of the shaft portion.

4. The femur plate of claim 3, wherein the receiving portion includes an elongated slot configured to extend across one or more of the plurality of holes of the shaft portion when the shaft and head portions are assembled together.

5. The femur plate of any one of claims 1 to 4, wherein one or more of the plurality of holes of the shaft and head permit polyaxial placement of screws.

6. The femur plate of any one of claims 1 to 5, wherein the shaft portion and the head portion are integral to each other.

7. The femur plate of claim 6, wherein the shaft portion includes an oblong hole disposed between two holes of the plurality of holes of the shaft portion.

8. The femur plate of any one of claims 1 to 7, wherein the plurality of holes of the two arms are configured to receive locking and non-locking screws.

9. The femur plate of any one of claims 1 to 8, wherein the two arms include bendable sections permitting bending of the arms.

10. The femur plate of any one of claims 1 to 9, wherein one of the two arms includes a prong extending from a terminal end thereof.

11. The femur plate of claim 10, wherein the prong is curved and includes a sharpened tip.

12. The femur plate of any one of claims 2 to 11, wherein the shaft portion and the head portion are attached by a screw extending through an elongated slot defined by the head portion.

13. The femur plate of any one of claims 1 to 12, further comprising a plurality of notches disposed in series within a groove in the head portion, and wherein each of the plurality of grooves is configured to receive a rib extending from a top surface of the shaft portion.

14. The femur plate of any one of claims 12 or 13 when dependent on 12, wherein the elongated slot of the head portion extends across a set of holes defined by the shaft portion when assembled together.

15. The femur plate of any one of claims 1 to 14, wherein the shaft portion defines a base plate and the head portion defines an attachment plate having an angled portion obliquely oriented with respect to a longitudinal axis of the base plate.
